# EUROPÄISCHE PATENTANMELDUNG

(11) **EP 3 570 060 A1**
(43) Veröffentlichungstag der Anmeldung: **20.11.2019**
(21) Anmeldenummer: 18172265.3
(22) Anmeldetag: 15.05.2018
(51) Int. Cl.: G01R 33/56, G01R 33/48

(54) **VERFAHREN ZUR REKONSTRUKTION VON MAGNETRESONANZTOMOGRAPHIE-AUFNAHMEN MIT VARIABLER ZEITAUFLÖSUNG**

(71) Anmelder: Siemens Healthcare GmbH, 91052 Erlangen (DE)
(72) Erfinder: Horger, Wilhelm, 90571 Schwaig (DE); Kartäusch, Ralf, 91058 Erlangen (DE); Paul, Dominik, 91088 Bubenreuth (DE)

(57) **Zusammenfassung**

Die Erfindung betrifft ein Verfahren zur Rekonstruktion von Magnetresonanztomographie-Aufnahmen mit variabler Zeitauflösung, umfassend die Schritte:
- Erstellen oder Bereitstellen eines Datensatzes umfassend eine Serie von Magnetresonanztomographie-Rohdaten (RD), welche in einem abgeschlossenen Zeitraum zu unterschiedlichen Zeiten von demselben Körperbereich eines Patienten (P) aufgenommen worden sind,
- Vor-Rekonstruktion von Vorschaubildern (VB) aus den Magnetresonanztomographie-Rohdaten (RD),
- Ermittlung von Messdaten (MD) aus den Vorschaubildern (VB) und/oder aus den Rohdaten (RD), welche die Anflutung eines Kontrastmittels über eine Zeitspanne quantitativ wiedergeben,
- Festlegung von Haupt-Rekonstruktionsparametern (HP) umfassend Zeitabschnitte (Z1, Z2), in denen mit unterschiedlichen Zeitauflösungen rekonstruiert wird, auf Basis der Messdaten (MD),
- Haupt-Rekonstruktion von Magnetresonanztomographie-Aufnahmen (B) aus den Magnetresonanztomographie-Rohdaten (RD), basierend auf den Haupt-Rekonstruktionsparametern (HP).

Die Erfindung betrifft des Weiteren eine Rekonstruktionsvorrichtung, eine entsprechende Steuereinrichtung und eine entsprechende Befundungsstation sowie ein medizintechnisches bildgebendes System.

## Beschreibung

Die Erfindung betrifft ein Verfahren zur Rekonstruktion von Magnetresonanztomographie-Aufnahmen mit variabler Zeitauflösung, eine diesbezügliche Rekonstruktionsvorrichtung eine entsprechende Steuereinrichtung und eine entsprechende Befundungsstation sowie ein medizintechnisches bildgebendes System.

Dynamische Vorgänge, insbesondere die Anflutung eines Kontrastmittels, mittels eines Magnetresonanztomographen zu messen ist aufgrund der vergleichsweise langen Aufnahmezeiten des technischen Systems mit Herausforderungen verbunden. Eine Möglichkeit bietet z.B. Compressed Sensing GRASP-VIBE.

Compressed Sensing GRASP-VIBE ist eine Technik die eine radiale Abtastung mit einer goldenen Winkelverteilung verwendet. Diese Technik wird üblicherweise eingesetzt um Dynamiken wie die Anflutung von Kontrastmitteln aufzunehmen. Das Akquisitionsschema erlaubt es Bilder zu beliebigen Zeitpunkten mit beliebiger Zeitauflösung zu rekonstruieren. Die Abkürzung "GRASP" steht dabei für "Golden-angle RAdial Sparse Parallel" und "VIBE" für "Volumetrie Interpolated Breath-hold/Brain Examination". Das Aufnahmeverfahren entspricht einer VIBE Sequenz mit radialer Trajektorie bei der eine kartesische Kodierung in die Schichtrichtung durchgeführt wird. Es werden kontinuierlich mit dem Abstand des goldenen Winkels k-Raum Speichen aufgenommen (siehe dazu auch "Golden-angle radial sparse parallel MRI: Combination of compressed sensing, parallel imaging, and golden-angle radial sampling for fast and flexible dynamic volumetric MRI", L. Feng et al., Magn Reson Med. 2014 Sep; 72(3):707-17).

Das GRASP-VIBE Verfahren kann dazu verwendet werden, die Anflutung eines Kontrastmittels zu messen. Die Dynamik der Kontrastmittelanflutung variiert dabei über die Dauer der Messung deutlich. Deshalb wird die Messung üblicherweise in unterschiedliche Phasen eingeteilt in denen die Zeitauflösung der zu erwartenden Dynamik angepasst werden muss. Beispielsweise sollte in der arteriellen Phase eine sehr hohe Zeitauflösung verwendet werden und in der sogenannten "späten Phase" bei der kaum noch Veränderungen stattfindet eine sehr geringe Zeitauflösung.

Die Zeitauflösung hat dabei direkt Auswirkungen auf die Qualität der rekonstruierten Bilder. Wählt man eine vergleichsweise niedrige Zeitauflösung, wird das Bild unschärfer. Wählt man eine vergleichsweise hohe Zeitauflösung, wird das Bild zwar schärfer aber das Rauschen und die für radiale Messungen aufgrund der stärkeren Unterabtastung üblichen Streaking Artefakte nehmen zu. Darüber hinaus soll die Zeitauflösung nicht konstant über die Messung hoch gewählt werden, da sonst sehr viele Bilder erstellt werden, die eine entsprechend lange Befundung bedingen.

Problematisch ist, dass die Zeitpunkte der Kontrastmittelanflutung je nach Läsion, Gefäß oder Organ deutlich variieren. Dadurch können die Start und Endzeitpunkte der Phasen unterschiedlicher Zeitauflösung nur grob geschätzt werden.

Um Phasen unterschiedlicher Zeitauflösung festzulegen, wird nach bisherigem Stand vor der Rekonstruktion entweder manuell ein Zeitpunkt der Anflutung abgeschätzt oder eine automatische Detektion der Anflutung des Kontrastmittels in der Aorta durchgeführt und in beiden Fällen die hohe Zeitauflösung zu den entsprechenden Zeitpunkten vorgegeben. Dabei wird nicht immer der optimale Zeitpunkt für manche Läsionen oder Gefäße getroffen, was einen großen Nachteil darstellt.

Es ist daher eine Aufgabe der vorliegenden Erfindung, die Nachteile des Standes der Technik zu überwinden und ein Verfahren und eine Rekonstruktionsvorrichtung zur verbesserten Rekonstruktion von Magnetresonanztomographie-Aufnahmen mit variabler Zeitauflösung, eine entsprechende Steuereinrichtung und eine entsprechende Befundungsstation sowie ein medizintechnisches bildgebendes System zur Verfügung zu stellen.

Diese Aufgabe wird zum einen durch ein Verfahren gemäß Patentanspruch 1, eine Rekonstruktionsvorrichtung gemäß Patentanspruch 8, eine Steuereinrichtung gemäß Patentanspruch 11, eine Befundungsstation gemäß Patentanspruch 12 sowie ein medizintechnisches bildgebendes System gemäß Patentanspruch 13 gelöst.

Das erfindungsgemäße Verfahren zur Rekonstruktion von Magnetresonanztomographie-Aufnahmen mit variabler Zeitauflösung, umfasst die folgenden Schritte:

### - Erstellen oder Bereitstellen von Rohdaten

In diesem Schritt wird ein neuer Datensatz umfassend Magnetresonanztomographie-Rohdaten erstellt. Es ist aber je nach Anwendungsfall auch möglich, auf einen bereits bestehenden Datensatz zurückzugreifen, z.B. durch Herunterladen einer entsprechenden Datei aus einem Datenspeicher und/oder Übermittlung des Datensatzes durch ein Netzwerk. Dieser Datensatz umfasst eine Serie von Rohdaten, welche mittels eines Magnetresonanztomographen in einem abgeschlossenen Zeitraum zu unterschiedlichen Zeiten von demselben Körperbereich eines Patienten aufgenommen worden sind. Dabei wurden die Magnetresonanztomographie-Rohdaten mittels eines Aufnahmeverfahrens erstellt, welches eine Rekonstruktion mit Sliding Window ermöglicht, also einer variablen Zeitauflösung, z.B. mittels eines GRASP- insbesondere GRASP-VIBE-Aufnahmeverfahrens.

### - Vor-Rekonstruktion von Vorschaubildern

In diesem Schritt werden Vorschaubilder aus den Magnetresonanztomographie-Rohdaten rekonstruiert. Dabei kann durchaus eine Rekonstruktion verwendet werden, wie sie bereits im Stand der Technik bekannt ist. Bezüglich der Anflutung eines Kontrastmittels können beispielsweise bekannte Voreinstellungen für unterschiedliche Zeitpunkte verwendet werden.

### - Ermittlung von Messdaten

In diesem Schritt werden aus den Vorschaubildern und/oder aus den Rohdaten (bevorzugt aus den Rohdaten) Messdaten ermittelt, welche die Anflutung eines Kontrastmittels über eine Zeitspanne quantitativ wiedergeben. Diese Messdaten sind z.B. die Daten einer GRASP-Messung, z.B. die Variation der Energie, bzw. der Signalintensität, im k-Raum Zentrum über die Zeit. Die Gegenwart eines Kontrastmittels sorgt für eine schnellere Relaxation, was sich in einer gesteigerten Energie äußert. Bevorzugt wird dazu im Vorschaubild ein Bereich ausgewählt, z.B. mittels eines dargestellten Fadenkreuzes bzw. einer "Pixel Lense". Aus den Messdaten lässt sich also entnehmen, welche Kontrastmittelkonzentration in einem Bereich zu welchem Zeitpunkt vorliegt.

Die Messdaten können zur Anschauung in Form einer Kontrastmittelkurve gebracht und angezeigt werden. Bei einer Kontrastmittelkurve wird gemäß dem Stand der Technik die Signalintensität im K-Raum Zentrum über die Zeit gefiltert aufgetragen. Bevorzugt wird dabei die Kontrastmittelkurve als grafische Auftragung der Messdaten über die Zeit bestimmt und besonders bevorzugt live angezeigt. Wurde ein Bereich ausgewählt (z.B. mittels eines Auswahlwerkzeugs, wie einer "Pixel Lense" in einem Vorschaubild), kann auch eine zweite (Vergleichs-)Kontrastmittelkurve auf Basis eines zweiten Bereichs (Vergleichsareal) erzeugt und beide Kontrastmittelkurven angezeigt werden.

### - Festlegung von Haupt-Rekonstruktionsparametern

Diese Haupt-Rekonstruktionsparameter dienen einer Rekonstruktion der aufgenommenen Rohdaten und umfassen Zeitabschnitte, in denen mit unterschiedlichen Zeitauflösungen rekonstruiert wird. Sie unterscheiden sich von den bei der Vor-Rekonstruktion verwendeten Parametern dadurch, dass Informationen, welche durch die Messdaten zusätzlich gewonnen worden sind, in sie eingegangen sind. Die Haupt-Rekonstruktionsparameter stellen somit gegenüber den bei der Vor-Rekonstruktion verwendeten Parametern verbesserte Parameter dar. Nach dem vorgenannten Beispiel ergeben sich die Haupt-Rekonstruktionsparameter bevorzugt basierend auf einer Kontrastmittelkurve, welche aus den Messdaten erstellt worden sind.

Die Haupt-Rekonstruktionsparameter können gemäß einer bevorzugten Ausführungsform manuell von einem Bediener festgelegt werden. Dabei wird dem Bediener die Möglichkeit gegeben, Zeitabschnitte basierend auf den Messdaten zu wählen. Dadurch kann der Bediener z.B. diejenigen Zeitabschnitte in den Messdaten auswählen, in denen eine hohe Dynamik herrscht. Bevorzugt kann der Bediener dafür über eine entsprechende Benutzerschnittstelle den Zeitpunkt anhand einer Kontrastmittelkurve wählen. Diese Auswahl kann aber auch automatisch z.B. aufgrund der Form einer Kontrastmittelkurve getroffen werden.

Man kann statt eines Zeitpunkts, der mit einer Zeitauflösung rekonstruiert wird, eine Zeitspanne wählen in der mehrere "Zeitpunkte" mit der gleichen Zeitauflösung rekonstruiert werden. Ein "Zeitpunkt" entspricht dabei einem 3D Volumen bzw. einem Schichtstapel mit von Bildern zu einem Zeitpunkt.

### - Haupt-Rekonstruktion

In diesem Schritt werden Magnetresonanztomographie-Aufnahmen, also für einen Befunder verständliche Bilder, aus den Magnetresonanztomographie-Rohdaten rekonstruiert. Die Rekonstruktion basiert dabei auf den vorangehend festgelegten Haupt-Rekonstruktionsparametern, also den dort vorliegenden Zeitabschnitten.

Bei der Haupt-Rekonstruktion wird also verglichen mit dem Stand der Technik nachträglich eine Rekonstruktion mit neuen Parametern (den Haupt-Rekonstruktionsparametern) gestartet. Es ist dazu anzumerken, dass eine Rekonstruktion auf den derzeitig verfügbaren Anlagen mit einer Rekonstruktionszeit von bis zu 5 min sehr lange dauert. Daher ist es vorzuziehen die Rekonstruktion auf die Schichten von Interesse zu begrenzen. Erstreckt sich eine Läsion z.B. nur auf wenige Schichten oder reicht eine Schicht aus um sie zu klassifizieren, kann die Rekonstruktion in wenigen Sekunden durchgeführt werden.

Zusammenfassend kann also gesagt werden, dass die Erfindung aus Vorschaubildern entnommene Informationen für eine Hauptrekonstruktion der Bilder verwendet.

Eine erfindungsgemäße Rekonstruktionsvorrichtung zur Rekonstruktion von Magnetresonanztomographie-Aufnahmen mit variabler Zeitauflösung, umfasst die folgenden Komponenten:
Eine Datenschnittstelle zur Erfassung eines Datensatzes umfassend eine Serie von Magnetresonanztomographie-Rohdaten, welche in einem abgeschlossenen Zeitraum zu unterschiedlichen Zeiten von demselben Körperbereich eines Patienten aufgenommen worden sind (s.o.).

Eine Vor-Rekonstruktionseinheit ausgelegt zur vorangehend beschriebenen Vor-Rekonstruktion von Vorschaubildern aus den Magnetresonanztomographie-Rohdaten.

Eine Ermittlungseinheit zur Ermittlung von Messdaten aus den Vorschaubildern und/oder aus den Rohdaten, welche die Anflutung eines Kontrastmittels über eine Zeitspanne quantitativ wiedergeben. Diese Ermittlungseinheit ist bevorzugt dazu ausgelegt eine Kontrastmittelkurve zu generieren und besonders bevorzugt auch auszugeben (zumindest die diesbezüglichen Daten), insbesondere in grafischer Form.

Eine Parametereinheit zur Festlegung von Haupt-Rekonstruktionsparametern umfassend Zeitabschnitte, in denen mit unterschiedlichen Zeitauflösungen rekonstruiert wird, auf Basis der Messwerte. Diese Parametereinheit kann mit einer Eingabeeinheit verbunden sein, welche eine manuelle Auswahl eines Befunders aufnimmt. Es ist aber auch möglich, dass die Parametereinheit aus Bereichen hoher Dynamik der Messdaten automatisch Zeitabschnitte bestimmt, in denen für eine Rekonstruktion unterschiedliche Zeitauflösungen verwendet werden sollen.

Eine Haupt-Rekonstruktionseinheit ausgelegt zur Haupt-Rekonstruktion von Magnetresonanztomographie-Aufnahmen aus den Magnetresonanztomographie-Rohdaten, basierend auf den Haupt-Rekonstruktionsparametern. Diese Haupt-Rekonstruktionseinheit kann bei einem praktischen Aufbau der Rekonstruktionsvorrichtung auch der Vor-Rekonstruktionseinheit entsprechen, es können also theoretisch dieselben Hardware- bzw. Softwaremodule verwendet werden. Bei der Hauptrekonstruktion muss aber bei der Rekonstruktion auf die Haupt-Rekonstruktionsparameter zurückgegriffen werden.

Bei entsprechend schneller Rekonstruktionshardware und Reduktion auf eine Schicht oder wenige Schichten (s.o.) kann eine entsprechende Benutzerschnittstelle zur manuellen Festlegung der Haupt-Rekonstruktionsparameter bevorzugt auch "Live"-Bilder (eventuell mit reduzierter Inplane-Auflösung) des jeweils aktuellen Zeitpunktes anzeigen, um den Benutzer eine optimale Auswahl eines gewünschten Zeitpunktes zu ermöglichen. Dieses Vorgehen führt auch zu neuen Möglichkeiten bei der ursprünglichen Vor-Rekonstruktion. Da die Bilder mit der hohen Dynamik erst im Nachhinein bei Interesse live erzeugt werden, kann die ursprüngliche Vor-Rekonstruktion mit deutlich niedriger Zeitauflösung vorgenommen werden. Dadurch wird diese schneller und die Anzahl der zu befundenen Bilder wird reduziert.

Ein Vorteil der Erfindung ist, dass für Läsionen, Gefäße oder Organe ein optimaler Kontrast erstellt werden kann. Das erfindungsgemäße Verfahren ermöglicht aber auch nachträglich Bilder zu optimieren, die durch Artefakte nur eingeschränkt diagnostizierbar sind. In einem beispielhaften Fall, in dem eine sehr hohe Zeitauflösung für die arterielle Phase gewählt wurde und durch die Physiologie bzw. Bewegung des Patienten Streaking Artefakte induziert wurden, kann nachträglich die Zeitauflösung verringert werden, was eine verbesserte Befundung ermöglicht. Wenn z.B. der Patient sich während der Messung bewegt, äußert sich das in Artefakten in den Bildern. Wählt man nun die Zeitpunkte so dass die Daten die während der Bewegung akquiriert wurden nicht verwendet werden, lassen sich die Artefakte entfernen.

Eine erfindungsgemäße Steuereinrichtung zur Steuerung eines Magnetresonanztomographiesystems umfasst eine erfindungsgemäße Rekonstruktionsvorrichtung und/oder ist für die Durchführung eines erfindungsgemäßen Verfahrens ausgestaltet.

Eine erfindungsgemäße Befundungsstation (beispielsweise eine leistungsfähige Rechenvorrichtung) ist mit einem Magnetresonanztomographiesystem gekoppelt oder koppelbar. Sie umfasst eine erfindungsgemäße Rekonstruktionsvorrichtung und/oder ist für die Durchführung eines erfindungsgemäßen Verfahrens ausgestaltet.

Ein erfindungsgemäßes medizintechnisches bildgebendes System umfasst eine erfindungsgemäße Rekonstruktionsvorrichtung und ein Magnetresonanztomographiesystem wobei die Rekonstruktionsvorrichtung im medizintechnischen bildgebenden System bevorzugt in Form einer erfindungsgemäßen Befundungsstation und/oder einer erfindungsgemäßen Steuereinrichtung vorliegt.

Ein Großteil der zuvor genannten Komponenten der Rekonstruktionsvorrichtung, der Befundungsstation bzw. der Steuereinrichtung, können ganz oder teilweise in Form von Softwaremodulen in einem Prozessor einer entsprechenden Rekonstruktionsvorrichtung, Befundungsstation bzw. Steuereinrichtung realisiert werden. Eine weitgehend softwaremäßige Realisierung hat den Vorteil, dass auch schon bisher verwendete Geräte auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Befundungsstation oder Steuereinrichtung eines Magnetresonanztomographiesystems ladbar ist, mit Programmabschnitten, um alle Schritte des erfindungsgemäßen Verfahrens auszuführen, wenn das Programm in dem Rechensystem, der Befundungsstation bzw. der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem, zur Befundungsstation bzw. zur Steuereinrichtung und/oder zur Speicherung an oder in dem Rechensystem, der Befundungsstation bzw. der Steuereinrichtung kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Befundungsstation bzw. Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt wird eine (große) Anzahl von Zwischenbildern rekonstruiert, insbesondere im Rahmen einer Vor-Rekonstruktion von Vorschaubildern. Diese Zwischenbilder sollten eine möglichst hohe Zeitauflösung haben (bevorzugt die maximal mögliche Zeitauflösung), wobei dadurch auftretende Artefakte zunächst nebensächlich sind. Bei der Haupt-Rekonstruktion werden dann diese bereits vorliegenden Zwischenbilder verwendet. Die Magnetresonanztomographie-Aufnahmen werden dabei durch Mittelung der Zwischenbilder gemäß der Haupt-Rekonstruktionsparameter rekonstruiert. Es werden also zunächst sehr viele Bilder erzeugt deren Anzahl bei der Haupt-Rekonstruktion durch die Mittelung auf die gewünschte Zeitauflösung wieder reduziert wird.

Bevorzugt erfolgt eine Anzeige der Messwerte, besonders bevorzugt in Form einer Kontrastmittelkurve, insbesondere zusammen mit Vorschaubildern. Eine bevorzugte Rekonstruktionsvorrichtung umfasst dazu eine Anzeigeeinheit zur Anzeige der Messdaten, vorzugsweise der Kontrastmittelkurve, insbesondere zusammen mit Vorschaubildern.

Bevorzugt erfolgt eine Wahl von Zeitabschnitten, und insbesondere auch eine Wahl der Zeitauflösung innerhalb der Zeitabschnitte, durch einen Benutzer, vorzugsweise auf Basis einer angezeigten Kontrastmittelkurve, insbesondere zusammen mit den angezeigten Vorschaubildern.

Zur Definition eines Zeitabschnitts ist bevorzugt ein Zeitpunkt (insbesondere bei einer vorher festgelegten Länge eines Zeitabschnitts) und bevorzugt zusätzlich eine Zeitspanne (insbesondere bei einer variablen Länge eines Zeitabschnitts) in den Haupt-Rekonstruktionsparametern enthalten. Es ist also möglich ggf. neben dem eigentlichen Zeitpunkt auch eine Zeitspanne zu wählen. Dadurch ergibt sich der Vorteil, dass zusätzlich auch der Grad der Unterabtastung bzw. Bildrauschen beeinflusst und optimiert werden kann.

Bevorzugt wird ein Zeitabschnitt mittels einer Bolus Detektion vorausgewählt.

Bevorzugt werden die Rohdaten mit einem GRASP-Aufnahmeverfahren (insbesondere radial vibe), bevorzugt mit einem Compressed Sensing GRASP-VIBE Aufnahmeverfahren aufgenommen.

Bevorzugt enthalten die Haupt-Rekonstruktionsparameter zusätzlich Informationen zu (für eine Untersuchung) relevanten Schichten, die mit einer bestimmten Zeitauflösung in einem bestimmten Zeitabschnitt rekonstruiert werden sollen. Basierend auf diesen Haupt-Rekonstruktionsparametern wird dann eine Haupt-Rekonstruktion der relevanten Schichten durchgeführt.

Bevorzugt umfasst die Rekonstruktionsvorrichtung eine Ausgabesteuereinheit ausgelegt zur Steuerung der Anzeigeeinheit dergestalt, dass eine Darstellung von grafischen Elementen erfolgt, welche eine Wahl von Zeitabschnitten, und insbesondere auch der Zeitauflösung innerhalb der Zeitabschnitte, durch einen Benutzer darstellen. Dabei ist die Ausgabesteuereinheit zusätzlich zu einer Anzeige von weiteren Vorschaubildern ausgelegt, die eine Vorabrekonstruktion von Magnetresonanztomographie-Aufnahmen darstellen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines Magnetresonanztomographiesystems gemäß einem Ausführungsbeispiel der Erfindung,
Figur 2 eine schematische Darstellung des erfindungsgemäßen Verfahrens,
Figur 3 ein Beispiel für ein Vorschaubild,
Figur 4 ein Beispiel für eine Kontrastmittelkurve.

In den folgenden Figuren sind nur für die Erfindung wesentliche oder zu ihrem Verständnis hilfreiche Elemente eingezeichnet.

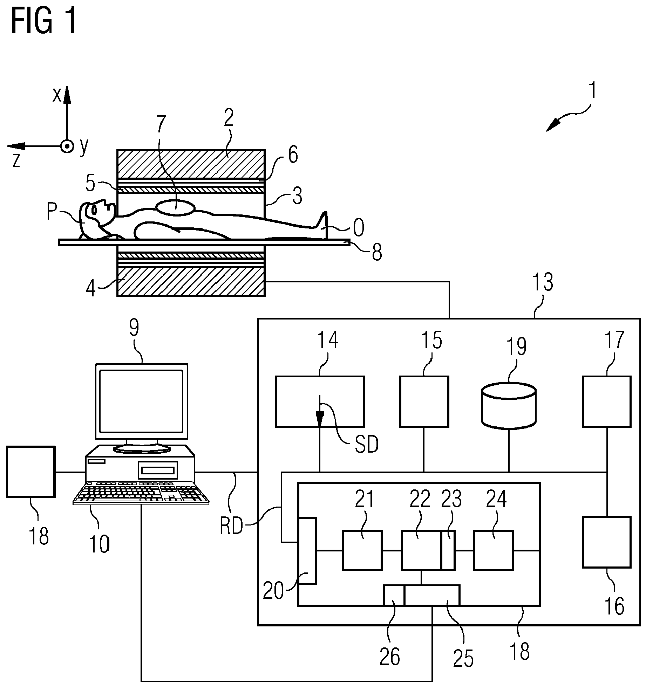

In Figur 1 ist grob schematisch ein Magnetresonanztomographiesystem 1 dargestellt. Es umfasst zum einen den eigentlichen Magnetresonanzscanner 2 mit einem Untersuchungsraum 3 bzw. Patiententunnel, in den auf einer Liege 8 ein Patient P oder Proband positioniert ist, in dessen Körper sich das eigentliche Untersuchungsobjekt O befindet.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennen-system 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht (in Figur 1 nur durch eine einzelne Lokalspule symbolisiert). Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem enthält der Magnetresonanzscanner 2 (nicht dargestellte) Shimspulen, die in üblicher Weise ausgebildet sein können.

Bei dem in Figur 1 dargestellten Magnetresonanztomographie-System handelt es sich um eine Ganzkörperanlage mit einem Patiententunnel, in den ein Patient P komplett eingebracht werden kann. Grundsätzlich kann die Erfindung aber auch an anderen Magnetresonanztomographie-Systemen, z. B. mit seitlich offenem, C-förmigen Gehäuse, verwendet werden. Wesentlich ist nur, dass entsprechende Aufnahmen des Untersuchungsobjekts O angefertigt werden können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz PS innerhalb einer Messsitzung gesteuert, so dass z.B. ein GRASP-VIBE-Aufnahmeverfahren angewandt wird. Üblicherweise sind verschiedene Steuerprotokolle mit Pulssequenzen PS für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden.

Zur Ausgabe der einzelnen HF-Pulse einer Pulssequenz PS weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz PS die Gradientenpulse passend zu schalten, weist die Steuereinrichtung 13 eine Gradientensystemschnittstelle 16 auf. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz PS.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz PS vorgegebenen Auslesefenster koordiniert mittels des HF-Empfangsantennensystems 7 Magnetresonanz-Signale zu empfangen und so die Rohdaten zu akquirieren.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Eine Rekonstruktionsvorrichtung 18 übernimmt hier die akquirierten Rohdaten RD und rekonstruiert daraus Magnetresonanz-Aufnahmen B im Rahmen der Erfindung. Diese Bilddaten können dann beispielsweise in dem Speicher 19 hinterlegt werden.

Die Rekonstruktionsvorrichtung 18 umfasst eine Datenschnittstelle 20 zur Erfassung eines Datensatzes mit einer Serie von Magnetresonanztomographie-Rohdaten RD, welche in einem abgeschlossenen Zeitraum zu unterschiedlichen Zeiten von demselben Körperbereich eines Patienten P aufgenommen worden sind. Diese Datenschnittstelle 20 steht mit einer Vor-Rekonstruktionseinheit 21 in Datenkontakt, welche dazu ausgelegt ist, eine Vor-Rekonstruktion der Magnetresonanztomographie-Rohdaten RD zu Vorschaubildern vorzunehmen.

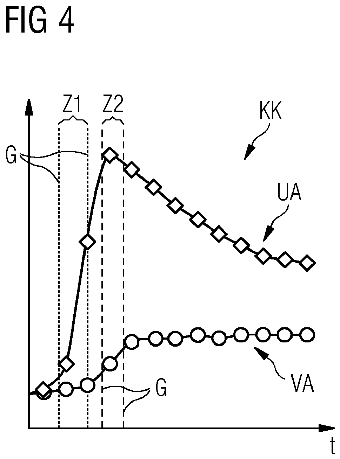

Eine Ermittlungseinheit 22 ermittelt Messdaten MD aus den Vorschaubildern VB und/oder aus den Rohdaten RD, welche die Anflutung eines Kontrastmittels über eine Zeitspanne quantitativ wiedergeben. Diese Messdaten können beispielsweise eine Kontrastmittelkurve KK wiedergeben, wie sie in Figur 4 gezeigt ist. Die Kontrastmittelkurve KK wird in diesem Beispiel zusammen mit einem Vorschaubild VB von einer Anzeigeeinheit 25 ausgegeben. Diese Anzeigeeinheit 25 kann wie in diesem Beispiel gezeigt als Schnittstelle vorliegen, welche eine Datenverbindung mit einem Terminal 10 herstellt. Sie kann aber auch ein eigenständiges Display beinhalten.

Die Rekonstruktionsvorrichtung umfasst in diesem Beispiel eine Ausgabesteuereinheit 26 zur Steuerung der Anzeigeeinheit 25. Die Ausgabesteuereinheit 26 erlaubt dabei eine Darstellung von grafischen Elementen, welche eine Wahl von Zeitabschnitten durch einen Benutzer darstellen. Sie kann aber auch zusätzlich zu einer Anzeige von weiteren Vorschaubildern VB ausgelegt ist, die eine Vorabrekonstruktion von Magnetresonanztomographie-Aufnahmen B darstellen.

Eine Parametereinheit 23 erlaubt eine Festlegung von Haupt-Rekonstruktionsparametern HP umfassend Zeitabschnitte, in denen mit unterschiedlichen Zeitauflösungen rekonstruiert wird, auf Basis der Messwerte. Basierend auf diesen Haupt-Rekonstruktionsparametern HP führt eine Haupt-Rekonstruktionseinheit 24 eine Haupt-Rekonstruktion von Magnetresonanztomographie-Aufnahmen B aus den Magnetresonanztomographie-Rohdaten RD durch.

Eine Bedienung der zentralen Steuereinrichtung 13 kann über ein Terminal 10 mit einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Aufnahmen B angezeigt werden, und mittels des Terminals 10 können Aufnahmen geplant und gestartet und insbesondere Steuerprotokolle P ausgewählt und gegebenenfalls modifiziert werden. Das Terminal 10 ist in diesem Fall als Befundungsstation 10 ausgestaltet und mit einer erfindungsgemäßen Rekonstruktionsvorrichtung 18 versehen, die z.B. wie vorangehend beschrieben aufgebaut ist.

Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk (und z.B. weiteren Befundungsstationen) zu verbinden und Rohdaten und/oder Bilddaten bzw. Parameterkarten, aber auch weitere Daten, wie beispielsweise patientenrelevante Daten oder Steuerprotokolle, austauschen zu können.

Wie durch ein Einstrahlen von HF-Pulsen und die Erzeugung von Gradientenfeldern geeignete Rohdaten akquiriert und daraus Magnetresonanztomographie-Bilder rekonstruiert werden können, ist dem Fachmann grundsätzlich bekannt und wird hier nicht näher erläutert. Ebenso sind verschiedenste Messsequenzen, wie z. B. EPI-Messsequenzen oder andere Messsequenzen zur Erzeugung von diffusionsgewichteten Bildern, dem Fachmann vom Grundsatz her bekannt.

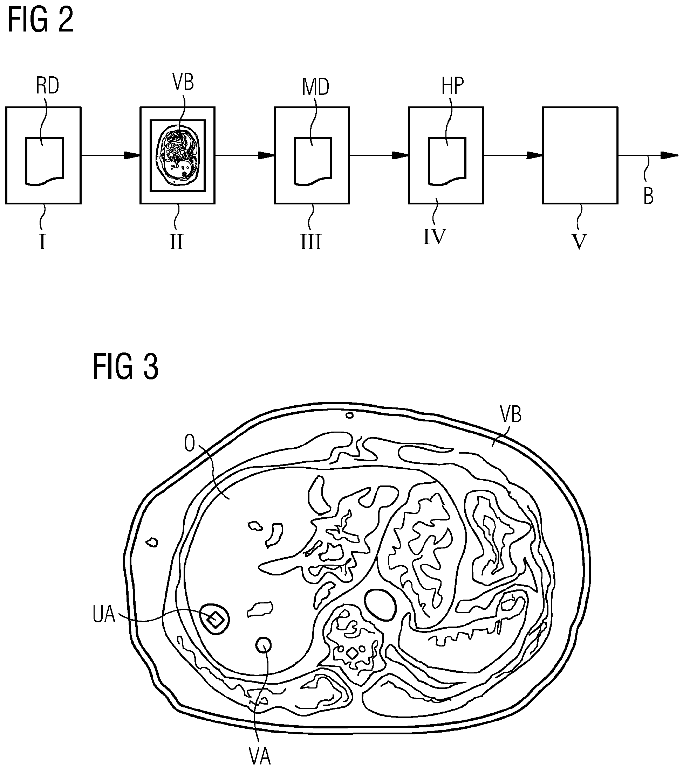

Figur 2 zeigt eine schematische Darstellung des erfindungsgemäßen Verfahrens zur Rekonstruktion von Magnetresonanztomographie-Aufnahmen mit variabler Zeitauflösung in Form eines Blockschaltbildes.

In Schritt I erfolgt eine Erstellung oder Bereitstellung eines Datensatzes umfassend eine Serie von Magnetresonanztomographie-Rohdaten RD, welche in einem abgeschlossenen Zeitraum zu unterschiedlichen Zeiten von demselben Körperbereich eines Patienten P, z.B. mittels eines GRASP-VIBE-Aufnahmeverfahrens, aufgenommen worden sind.

In Schritt II erfolgt eine Vor-Rekonstruktion von Vorschaubildern VB aus den Magnetresonanztomographie-Rohdaten RD.

In Schritt III erfolgt eine Ermittlung von Messdaten MD aus den Vorschaubildern VB und/oder aus den Rohdaten RD, welche die Anflutung eines Kontrastmittels über eine Zeitspanne quantitativ wiedergeben. Aus diesen Messdaten kann eine Kontrastmittelkurve KK erstellt werden, welche angezeigt werden kann.

In Schritt IV erfolgt auf Basis der Messdaten MD eine Festlegung von Haupt-Rekonstruktionsparametern HP umfassend Zeitabschnitte, in denen mit unterschiedlichen Zeitauflösungen rekonstruiert wird.

Die Haupt-Rekonstruktionsparameter HP können beispielsweise manuell festgelegt werden. Dazu erfolgt beispielsweise eine Anzeige der Messdaten MD in Form einer Kontrastmittelkurve KK zusammen mit einem Vorschaubild VB Eine solche Anzeige ist z.B. in den Figuren 3 und 4 zu sehen.

Ein Befunder kann beispielsweise mittels einer grafischen Oberfläche, auf der bewegliche Auswahlinstrumente dargestellt sind, die Haupt-Rekonstruktionsparameter HP festlegen.

In Schritt V erfolgt eine Haupt-Rekonstruktion von Magnetresonanztomographie-Aufnahmen B aus den Magnetresonanztomographie-Rohdaten RD, basierend auf den Haupt-Rekonstruktionsparametern HP.

Figur 3 zeigt ein Beispiel für ein Vorschaubild. Gezeigt ist hier ein Schnittbild durch eine menschliche Leber, die das Untersuchungsobjekt O darstellt. In dieser Leber ist ein Bereich einer Läsion eingezeichnet (umrandet), in dem ein Untersuchungsareal UA eingezeichnet ist. In diesem Untersuchungsareal UA ist es von Interesse, wann ein Kontrastmittel anflutet. Zum Vergleich werden auch Daten eines Vergleichsareals VA dargestellt.

Figur 4 zeigt ein Beispiel für eine Kontrastmittelkurve KK. In einem Koordinatensystem, in dem die X-Achse der Zeitachse t entspricht ist über die Zeit die gemessene Kontrastmittelmenge im Untersuchungsareal UA und zum Vergleich auch die des Vergleichsareals jeweils auf der Y-Achse eingetragen. Deutlich weicht der Verlauf in der Läsion (also der des Untersuchungsareals UA) von dem im gesunden Gewebe ab. Ein Befunder kann nun mittels beweglicher Grenzanzeigen G Zeitabschnitte Z1, Z2 eingrenzen, in denen eine vorbestimmte Zeitauflösung bei der Rekonstruktion angewandt werden soll. In diesem Beispiel existieren zwei Zeitabschnitte Z1, Z2, die durch unterschiedliche Grenzanzeigen G markiert sind (unterschiedliche Strichelung) In einem ersten Zeitabschnitt Z1 soll mit einer höheren Zeitauflösung rekonstruiert werden als in dem zweiten Zeitabschnitt Z2. Ein Befunder kann beispielsweise die Grenzanzeigen G auf der Zeitachse t verschieben und so gezielt diejenigen Zeitabschnitte Z1, Z2 in der Kontrastmittelkurve KK auswählen in denen mit der gewünschten Zeitauflösung rekonstruiert werden soll.

Es wird abschließend noch einmal darauf hingewiesen, dass es sich bei den vorhergehend detailliert beschriebenen Verfahren sowie bei dem dargestellten Magnetresonanztomographiesystem 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Rekonstruktion von Magnetresonanztomographie-Aufnahmen mit variabler Zeitauflösung, umfassend die Schritte:
- Erstellen oder Bereitstellen eines Datensatzes umfassend eine Serie von Magnetresonanztomographie-Rohdaten (RD), welche in einem abgeschlossenen Zeitraum zu unterschiedlichen Zeiten von demselben Körperbereich eines Patienten (P) aufgenommen worden sind,
- Vor-Rekonstruktion von Vorschaubildern (VB) aus den Magnetresonanztomographie-Rohdaten (RD),
- Ermittlung von Messdaten (MD) aus den Vorschaubildern (VB) und/oder aus den Rohdaten (RD), welche die Anflutung eines Kontrastmittels über eine Zeitspanne quantitativ wiedergeben,
- Festlegung von Haupt-Rekonstruktionsparametern (HP) umfassend Zeitabschnitte (Z1, Z2), in denen mit unterschiedlichen Zeitauflösungen rekonstruiert wird, auf Basis der Messdaten (MD),
- Haupt-Rekonstruktion von Magnetresonanztomographie-Aufnahmen (B) aus den Magnetresonanztomographie-Rohdaten (RD), basierend auf den Haupt-Rekonstruktionsparametern (HP).

2. Verfahren nach Anspruch 1, wobei im Rahmen der Vor-Rekonstruktion zunächst eine Rekonstruktion von Zwischenbildern (ZB) mit der maximal möglichen Zeitauflösung durchgeführt wird und bei der Haupt-Rekonstruktion die Magnetresonanztomographie-Aufnahmen (B) durch Mittelung der Zwischenbilder (ZB) gemäß der Haupt-Rekonstruktionsparameter (RP) rekonstruiert werden.

3. Verfahren nach einem der vorangehenden Ansprüche, wobei eine Anzeige der Messdaten (MD), besonders bevorzugt in Form einer Kontrastmittelkurve (KK), insbesondere zusammen mit Vorschaubildern (VB), auf einer Anzeigeeinheit (25) erfolgt.

4. Verfahren nach einem der vorangehenden Ansprüche, wobei eine Wahl von Zeitabschnitten (Z1, Z2) durch einen Benutzer erfolgt, bevorzugt auf Basis einer angezeigten Kontrastmittelkurve, insbesondere zusammen mit den angezeigten Vorschaubildern.

5. Verfahren nach einem der vorangehenden Ansprüche, wobei zur Definition eines Zeitabschnitts (Z1, Z2) ein Zeitpunkt und bevorzugt zusätzlich eine Zeitspanne in den Haupt-Rekonstruktionsparametern (HP) enthalten ist.

6. Verfahren nach einem der vorangehenden Ansprüche, wobei die Rohdaten mit einem GRASP-Aufnahmeverfahren, bevorzugt mit einem Compressed Sensing GRASP-VIBE Aufnahmeverfahren aufgenommen werden.

7. Verfahren nach einem der vorangehenden Ansprüche, wobei die Haupt-Rekonstruktionsparameter (HP) zusätzlich Informationen zu relevanten Schichten umfassen, die mit einer bestimmten Zeitauflösung in einem bestimmten Zeitabschnitt (Z1, Z2) rekonstruiert werden sollen und eine Haupt-Rekonstruktion der relevanten Schichten durchgeführt wird.

8. Rekonstruktionsvorrichtung (18) zur Rekonstruktion von Magnetresonanztomographie-Aufnahmen mit variabler Zeitauflösung, umfassend:
- eine Datenschnittstelle (20) zur Erfassung eines Datensatzes umfassend eine Serie von Magnetresonanztomographie-Rohdaten (RD), welche in einem abgeschlossenen Zeitraum zu unterschiedlichen Zeiten von demselben Körperbereich eines Patienten (P) aufgenommen worden sind,
- eine Vor-Rekonstruktionseinheit (21) ausgelegt zur Vor-Rekonstruktion von Vorschaubildern (VB) aus den Magnetresonanztomographie-Rohdaten (RD),
- ein Ermittlungseinheit (22) zur Ermittlung von Messdaten (MD) aus den Vorschaubildern (VB) und/oder aus den Rohdaten (RD), welche die Anflutung eines Kontrastmittels über eine Zeitspanne quantitativ wiedergeben,
- eine Parametereinheit (23) zur Festlegung von Haupt-Rekonstruktionsparametern (HP) umfassend Zeitabschnitte (Z1, Z2), in denen mit unterschiedlichen Zeitauflösungen rekonstruiert wird, auf Basis der Messdaten (MD),
- eine Haupt-Rekonstruktionseinheit (24) ausgelegt zur Haupt-Rekonstruktion von Magnetresonanztomographie-Aufnahmen (B) aus den Magnetresonanztomographie-Rohdaten (RD), basierend auf den Haupt-Rekonstruktionsparametern (HP).

9. Rekonstruktionsvorrichtung (18) nach Anspruch 8 umfassend eine Anzeigeeinheit (25) zur Anzeige der Kontrastmittelkurve (KK), insbesondere zusammen mit Vorschaubildern (VB).

10. Rekonstruktionsvorrichtung (18) nach Anspruch 9 umfassend eine Ausgabesteuereinheit (26) ausgelegt zur Steuerung der Anzeigeeinheit (25) dergestalt, dass eine Darstellung von grafischen Elementen erfolgt, welche eine Wahl von Zeitabschnitten (Z1, Z2) durch einen Benutzer darstellen, wobei bevorzugt die Ausgabesteuereinheit (26) zusätzlich zu einer Anzeige von weiteren Vorschaubildern (VB) ausgelegt ist, die eine Vorabrekonstruktion von Magnetresonanztomographie-Aufnahmen (B) darstellen.

11. Steuereinrichtung (13) zur Steuerung eines Magnetresonanztomographiesystems (1), welche eine Rekonstruktionsvorrichtung (18) nach einem der Ansprüche 8 bis 10 umfasst und/oder zur Durchführung eines Verfahrens nach einem der Ansprüche 1 bis 7 ausgestaltet ist.

12. Befundungsstation (10) gekoppelt oder koppelbar mit einem Magnetresonanztomographiesystem (1), welche eine Rekonstruktionsvorrichtung (18) nach einem der Ansprüche 8 bis 10 umfasst und/oder zur Durchführung eines Verfahrens nach einem der Ansprüche 1 bis 7 ausgestaltet ist.

13. Medizintechnisches bildgebendes System (1) umfassend eine Rekonstruktionsvorrichtung (18) nach einem der Ansprüche 8 bis 10 und ein Magnetresonanztomographiesystem (1) wobei die Rekonstruktionsvorrichtung (18) im medizintechnischen bildgebenden System (1) bevorzugt in Form einer Befundungsstation (10) nach Anspruch 12 und/oder einer Steuereinrichtung (13) nach Anspruch 11 vorliegt.

14. Computerprogrammprodukt mit einem Computerprogramm, welches direkt in eine Speichereinrichtung eines Rechensystems, einer Befundungsstation (10) oder einer Steuereinrichtung (13) eines medizintechnischen bildgebenden Systems (1) ladbar ist, mit Programmabschnitten, um alle Schritte des Verfahrens nach einem der Ansprüche 1 bis 7 auszuführen, wenn das Computerprogramm in dem Rechensystem, der Befundungsstation (10) oder der Steuereinrichtung (13) ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 7 auszuführen, wenn die Programmabschnitte von der Rechnereinheit ausgeführt werden.

## Geänderte Patentansprüche

### Geänderte Patentansprüche gemäss Regel 137(2) EPÜ.

1. Verfahren zur Rekonstruktion von Magnetresonanztomographie-Aufnahmen mit variabler Zeitauflösung, umfassend die Schritte:
- Erstellen oder Bereitstellen eines Datensatzes umfassend eine Serie von Magnetresonanztomographie-Rohdaten (RD), welche in einem abgeschlossenen Zeitraum zu unterschiedlichen Zeiten von demselben Körperbereich eines Patienten (P) aufgenommen worden sind,
- Vor-Rekonstruktion von Vorschaubildern (VB) aus den Magnetresonanztomographie-Rohdaten (RD),
- Ermittlung von Messdaten (MD) aus den Vorschaubildern (VB) und/oder aus den Rohdaten (RD), welche die Anflutung eines Kontrastmittels über eine Zeitspanne quantitativ wiedergeben,
- Festlegung von Haupt-Rekonstruktionsparametern (HP) umfassend Zeitabschnitte (Z1, Z2), in denen mit unterschiedlichen Zeitauflösungen rekonstruiert wird, auf Basis der Messdaten (MD),
- Haupt-Rekonstruktion von Magnetresonanztomographie-Aufnahmen (B) aus den Magnetresonanztomographie-Rohdaten (RD), basierend auf den Haupt-Rekonstruktionsparametern (HP), also den dort vorliegenden Zeitabschnitten.

2. Verfahren nach Anspruch 1, wobei im Rahmen der Vor-Rekonstruktion zunächst eine Rekonstruktion von Zwischenbildern (ZB) mit der maximal möglichen Zeitauflösung durchgeführt wird und bei der Haupt-Rekonstruktion die Magnetresonanztomographie-Aufnahmen (B) durch Mittelung der Zwischenbilder (ZB) gemäß der Haupt-Rekonstruktionsparameter (RP) rekonstruiert werden.

3. Verfahren nach einem der vorangehenden Ansprüche, wobei eine Anzeige der Messdaten (MD), besonders bevorzugt in Form einer Kontrastmittelkurve (KK), insbesondere zusammen mit Vorschaubildern (VB), auf einer Anzeigeeinheit (25) erfolgt.

4. Verfahren nach einem der vorangehenden Ansprüche, wobei eine Wahl von Zeitabschnitten (Z1, Z2) durch einen Benutzer erfolgt, bevorzugt auf Basis einer angezeigten Kontrastmittelkurve, insbesondere zusammen mit den angezeigten Vorschaubildern.

5. Verfahren nach einem der vorangehenden Ansprüche, wobei zur Definition eines Zeitabschnitts (Z1, Z2) ein Zeitpunkt und bevorzugt zusätzlich eine Zeitspanne in den Haupt-Rekonstruktionsparametern (HP) enthalten ist.

6. Verfahren nach einem der vorangehenden Ansprüche, wobei die Rohdaten mit einem GRASP-Aufnahmeverfahren, bevorzugt mit einem Compressed Sensing GRASP-VIBE Aufnahmeverfahren aufgenommen werden.

7. Verfahren nach einem der vorangehenden Ansprüche, wobei die Haupt-Rekonstruktionsparameter (HP) zusätzlich Informationen zu relevanten Schichten umfassen, die mit einer bestimmten Zeitauflösung in einem bestimmten Zeitabschnitt (Z1, Z2) rekonstruiert werden sollen und eine Haupt-Rekonstruktion der relevanten Schichten durchgeführt wird.

8. Rekonstruktionsvorrichtung (18) zur Rekonstruktion von Magnetresonanztomographie-Aufnahmen mit variabler Zeitauflösung, umfassend:
- eine Datenschnittstelle (20) zur Erfassung eines Datensatzes umfassend eine Serie von Magnetresonanztomographie-Rohdaten (RD), welche in einem abgeschlossenen Zeitraum zu unterschiedlichen Zeiten von demselben Körperbereich eines Patienten (P) aufgenommen worden sind,
- eine Vor-Rekonstruktionseinheit (21) ausgelegt zur Vor-Rekonstruktion von Vorschaubildern (VB) aus den Magnetresonanztomographie-Rohdaten (RD),
- ein Ermittlungseinheit (22) zur Ermittlung von Messdaten (MD) aus den Vorschaubildern (VB) und/oder aus den Rohdaten (RD), welche die Anflutung eines Kontrastmittels über eine Zeitspanne quantitativ wiedergeben,
- eine Parametereinheit (23) zur Festlegung von Haupt-Rekonstruktionsparametern (HP) umfassend Zeitabschnitte (Z1, Z2), in denen mit unterschiedlichen Zeitauflösungen rekonstruiert wird, auf Basis der Messdaten (MD),
- eine Haupt-Rekonstruktionseinheit (24) ausgelegt zur Haupt-Rekonstruktion von Magnetresonanztomographie-Aufnahmen (B) aus den Magnetresonanztomographie-Rohdaten (RD), basierend auf den Haupt-Rekonstruktionsparametern (HP), also den dort vorliegenden Zeitabschnitten.

9. Rekonstruktionsvorrichtung (18) nach Anspruch 8 umfassend eine Anzeigeeinheit (25) zur Anzeige der Kontrastmittelkurve (KK), insbesondere zusammen mit Vorschaubildern (VB).

10. Rekonstruktionsvorrichtung (18) nach Anspruch 9 umfassend eine Ausgabesteuereinheit (26) ausgelegt zur Steuerung der Anzeigeeinheit (25) dergestalt, dass eine Darstellung von grafischen Elementen erfolgt, welche eine Wahl von Zeitabschnitten (Z1, Z2) durch einen Benutzer darstellen, wobei bevorzugt die Ausgabesteuereinheit (26) zusätzlich zu einer Anzeige von weiteren Vorschaubildern (VB) ausgelegt ist, die eine Vorabrekonstruktion von Magnetresonanztomographie-Aufnahmen (B) darstellen.

11. Steuereinrichtung (13) zur Steuerung eines Magnetresonanztomographiesystems (1), welche eine Rekonstruktionsvorrichtung (18) nach einem der Ansprüche 8 bis 10 umfasst und/oder zur Durchführung eines Verfahrens nach einem der Ansprüche 1 bis 7 ausgestaltet ist.

12. Befundungsstation (10) gekoppelt oder koppelbar mit einem Magnetresonanztomographiesystem (1), welche eine Rekonstruktionsvorrichtung (18) nach einem der Ansprüche 8 bis 10 umfasst und/oder zur Durchführung eines Verfahrens nach einem der Ansprüche 1 bis 7 ausgestaltet ist.

13. Medizintechnisches bildgebendes System (1) umfassend eine Rekonstruktionsvorrichtung (18) nach einem der Ansprüche 8 bis 10 und ein Magnetresonanztomographiesystem (1) wobei die Rekonstruktionsvorrichtung (18) im medizintechnischen bildgebenden System (1) bevorzugt in Form einer Befundungsstation (10) nach Anspruch 12 und/oder einer Steuereinrichtung (13) nach Anspruch 11 vorliegt.

14. Computerprogrammprodukt mit einem Computerprogramm, welches direkt in eine Speichereinrichtung eines Rechensystems, einer Befundungsstation (10) oder einer Steuereinrichtung (13) eines medizintechnischen bildgebenden Systems (1) ladbar ist, mit Programmabschnitten, um alle Schritte des Verfahrens nach einem der Ansprüche 1 bis 7 auszuführen, wenn das Computerprogramm in dem Rechensystem, der Befundungsstation (10) oder der Steuereinrichtung (13) ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 7 auszuführen, wenn die Programmabschnitte von der Rechnereinheit ausgeführt werden.
